# EUROPÄISCHE PATENTANMELDUNG

(11) **EP 2 636 392 A1**
(43) Veröffentlichungstag der Anmeldung: **11.09.2013**
(21) Anmeldenummer: 13156766.1
(22) Anmeldetag: 26.02.2013
(51) Int. Cl.: A61F 11/00, A61H 9/00, A61M 13/00

(54) **Vorrichtung zur Tinnitusbehandlung**

(30) Priorität: 07.03.2012 DE 102012101917
(71) Anmelder: Veitl, Christian, 82544 Egling (DE)
(72) Erfinder: Veitl, Christian, 82544 Egling (DE)
(74) Vertreter: Kuhnen & Wacker

(57) **Zusammenfassung**

Die Erfindung betrifft eine Vorrichtung (1) zur Tinnitusbehandlung mit einer Bedienkonsole (2) mit einer Bedienoberfläche und mit wenigstens einem mit der Bedienkonsole (2) verbundenen Ohrstöpsel (36) zur Einbringung eines Tonmusters aus Tönen mit Tonhöhen in einer Frequenz von mehr als 30 Hz in den äußeren Gehörgang des Ohres. Diese Vorrichtung (1) zeichnet sich insbesondere dadurch aus, dass der Ohrstöpsel (36) über einen Druckschlauch (5) mit einem Druckerzeuger verbunden ist, der Druckpulsationen mit einer Pulsationsfrequenz von weniger als 10 Hz und einer Druckdifferenz von weniger als 1 bar erzeugt und über den Druckschlauch (5) in den äußeren Gehörgang des Ohres zuführt. Damit ist es möglich, eine Vorrichtung (1) zur Tinnitusbehandlung zu schaffen, mit der bessere Behandlungserfolge für einen größeren Personenkreis der Betroffenen erzielbar sind.

## Beschreibung

Die Erfindung betrifft eine Vorrichtung zur Tinnitusbehandlung mit einer Bedienkonsole mit einer Bedienoberfläche und mit wenigstens einem mit der Bedienkonsole verbundenen Ohrstöpsel zur Einbringung eines Tonmusters aus Tönen mit Tonhöhen in einer Frequenz von mehr als 30 Hz in den äußeren Gehörgang eines Ohres.

Unter Tinnitus versteht man ein Phantomgeräusch, welches eine Person subjektiv wahrnimmt, obwohl andere Personen in der Umgebung dieses Geräusch nicht hören können. Dieses Tinnitusgeräusch kann zu einer schweren Belastung für die betroffene Person werden und insbesondere zu Konzentrationsproblemen, Schlafstörungen, Depressionen oder sogar zu Angstzuständen führen. In der medizinischen Behandlung gibt es bislang verschiedene Ansätze, welche sich in der Regel auf das Innenohr, den Hörnerv oder das Hörzentrum in der Großhirnrinde, dem sogenannten auditorischen Cortex, konzentrieren. Dementsprechend gibt es bislang auch bereits verschiedene Behandlungsmethoden, welche sich jeweils auf einen dieser Bereiche konzentrieren.

Ein Beispiel hierfür findet sich in der DE 690 24 408 T2, welche eine Vorrichtung zur Regulierung des Drucks im äußeren Gehörgang einer Person offenbart. Diese Vorrichtung weist einen Körper auf, der so bemessen und geformt ist, dass er in den äußeren Gehörgang der Person eingesetzt werden kann. Er besitzt eine Öffnung, die den Transport von Fluiden in und aus dem äußeren Gehörgang bei hierin eingesetzter Vorrichtung ermöglicht, um einen Druck hierin zu erzeugen. Dabei wird durch diese Vorrichtung ein ausgewählter statischer Druck erzeugt, der einen niedrigeren Absolutwert besitzt als der Umgebungsdruck. Durch diese Druckbehandlung soll wenigstens eine Verminderung oder Unterdrückung des Tinnitus herbeigeführt werden. Eine nachhaltige Beseitigung des Tinnitus ist damit jedoch nicht gelungen; zudem erwies sich diese Vorrichtung als nur für einen begrenzten Personenkreis hilfreich.

Einen anderen Weg bestreitet die Firma ANM Adaptive Neuromodulation GmbH mit Ihrer akustischen CR^{®}-Neuromodulation. Diese Behandlungsmethode sieht die Ursache für das Phantomgeräusch in krankhaft synchronisierten, überaktiven Nervenzellverbänden im Gehirn. Ganz gezielt wird dabei die räumliche Anordnung der Tonfrequenzen im auditorischen Cortex genutzt, um die akustische Stimulation im betroffenen Areal durchzuführen. Abgestimmt auf eine vorab zu bestimmende Tinnitusfrequenz des Patienten werden mit Hilfe eines speziellen Algorhythmus passende Impulse berechnet und dann in zeitlicher Koordination zueinander programmiert. Hierdurch soll die synchrone Tinnitusaktivität im auditorischen Cortex gestört werden. Die Stimulation findet dabei über medizinische Kopfhörer statt. Durch diese Desynchronisierung der Nervenzellen soll das krankhafte Verhalten der Nervenzellen aufgehoben werden.

Auch diese Behandlungsmethode leidet daran, dass die tatsächlichen Ursachen für einen Tinnitus nicht abschließend erforscht sind und zudem unterschiedlich sein können. Durch die hier vorgeschlagene Stimulation der Nervenzellen mit einem Tonmuster kann daher nur sehr begrenzt Patienten geholfen werden. Tatsächlich hat sich in der Praxis gezeigt, dass jeder Tinnitus sehr subjektiv ist, bei jedem Menschen seine eigene Ausprägung hat und auf individuellen Ursachen beruht. Insofern mangelt es weiterhin an einem ganzheitlichen Ansatz zur Tinnitusbehandlung, welcher zudem für eine möglichst große Gruppe an Betroffenen geeignet ist.

Der Erfindung liegt daher die Aufgabe zu Grunde, eine Vorrichtung zur Tinnitusbehandlung zu schaffen, welche bessere Behandlungserfolge für einen größeren Personenkreis der Betroffenen erlaubt.

Diese Aufgabe wird durch eine Vorrichtung mit den Merkmalen des Anspruches 1 gelöst. Diese zeichnet sich insbesondere dadurch aus, dass der Ohrstöpsel über einen Druckschlauch mit einem Druckerzeuger verbunden ist, der Druckpulsationen mit einer Pulsationsfrequenz von weniger als 10 Hz und einer Druckdifferenz von weniger als 1 bar erzeugt und über den Druckschlauch in den äußeren Gehörgang des Ohres zuführt.

Der Erfindung liegt die Erkenntnis zu Grunde, dass für eine erfolgreiche Behandlung von Tinnitus eine Betrachtung aller Wirkzusammenhänge sowie eine vollständige Erfassung der betroffenen Bereiche im Körper erforderlich ist. So betrifft ein Tinnitus im Wesentlichen vier Bereiche: Das physikalische Hörorgan bzw. Ohr, die Nervenleitung, die physikalische Gehirnfunktion und die logische Gehirnfunktion.

Das Ohr ist das offensichtlich betroffene Organ bei einem Tinnitus. Die ältesten Analysen und Forschungsergebnisse zum Tinnitus konzentrieren sich fast vollständig auf das Ohr. Nach dem heutigen Forschungsstand spricht vieles dafür, dass ein Tinnitus im Ohr verschiedene Ursachen oder Symptome haben kann: So kann ein Tinnitus hier durch ein Trauma, eine Beschädigung oder Verletzung beispielsweise durch zu lauten Schall oder Aufprall, eine Druckwelle etc. hervorgerufen werden. Alternativ ist es auch möglich, dass eine Entzündung einen Tinnitus hervorruft oder eine Durchblutungsstörung durch Verengung oder Verstopfung, bzw. Versteifung oder Verhärtung von Gefäßen die Ursache für das Ohrgeräusch ist.

Nach dem aktuellen Forschungsstand liegen die im Ohr betroffenen Bereiche dabei im Innenohr. In der Hörschnecke befinden sich die Haarzellen oder Stereozilien, die von den genannten Ursachen beeinflusst bzw. beschädigt werden. Sie bilden die Schnittstelle zu den Nervenzellen bzw. zur Nervenleitung. Bereits bei der Betrachtung des Ohres und den Ursachen und Zusammenhängen mit dem Tinnitus ist festzustellen, dass häufig mehrere Symptome oder Ursachen vorliegen können, wobei der genaue Zusammenhang zwischen Ursache und Symptom nur schwer feststellbar ist.

Ein weiterer betroffener Bereich ist der Hörnerv, der den Hörimpuls des Hörorgans an das Gehirn weiterleitet. Als eine Ursache oder ein Symptom bei Tinnitus wird die Überreizung und/oder Entzündung des Hörnervs angesehen.

Ferner ist auch die physikalische Gehirnfunktion betroffen. Der im Gehirn relevante Bereich ist der auditorische Cortex, der seitlich im Schläfenlappen liegt und den über die Hörnerven weitergeleiteten Hörimpuls verarbeitet. Nach dem derzeitigen wissenschaftlichen Kenntnisstand beeinflusst ein Tinnitus diesen Bereich massiv. Möglicherweise findet sich hier sogar die Ursache des Tinnitus.

Darüber hinaus ist auch die logische Gehirnfunktion von Bedeutung. So wurde bereits früh erkannt, dass ein Tinnitus nicht nur durch physikalische Ereignisse verursacht wird, sondern vor allem auch mentale Aspekte eine besondere Rolle spielen. So ist Stress als eine Ursache von Tinnitus bekannt.

Im Rahmen der Erfindung wurde nun erkannt, dass für eine vollständige Heilung von Tinnitus das Zusammenwirken all dieser betroffenen Bereiche berücksichtigt werden muss. Dabei sind die komplexen Wirkzusammenhänge zu berücksichtigen, zumal bei einem chronischen Tinnitus auch gleichzeitig alle vier Bereiche im Körper akut betroffen sind. Zudem lässt sich dann in der Regel nicht mehr feststellen, was die eigentliche Ursache des Tinnitus war, und was nun Symptom ist. Erfindungsgemäß wurde erkannt, dass die Symptome auch die Rolle der Ursache erfüllen, sobald sie sich ausgeprägt haben. Dann wird das Symptom zur neuen Ursache und die Ursache wird zum Symptom.

Dies wird an einem konkreten Beispiel deutlich: So kann Stress zur Überreizung des auditorischen Cortex und der Hörnerven führen, oder bis hin zur Entzündung und Durchblutungsstörung des Hörorgans. Umgekehrt kann ein Trauma im Hörorgan dazu führen, dass der auditorische Cortex und die Hörnerven überreizt werden und dadurch bis hin zu Stress und Depressionen führen. Diese Wechselwirkungen sind vielfältig und auch bereits durch Studien belegt.

Durch die erfindungsgemäße Vorrichtung werden nun erstmals alle betroffenen Bereiche gleichermaßen behandelt. Die Erfindung nutzt dabei folgende Behandlungskomponenten: Luftdruckmassage der Hörorgane, akustische Massage der Hörorgane, Stimulation der Hörnerven und des akustischen Cortex und eine mentale Entspannung. In praktischen Versuchen hat sich dabei gezeigt, dass durch das kombinatorische Zusammenwirken dieser Behandlungskomponenten besonders gute Heilungserfolge erzielt werden können.

Dabei ist die Heilwirkung der einzelnen Behandlungskomponenten von wesentlicher Bedeutung: So stellt die Luftdruckmassage der Hörorgane eine physische Massage dar, welche verhärtete und steife Gefäße in Bewegung bringt. Sie fördert dadurch die Durchblutung und Mobilität und wirkt beruhigend auf den Patienten.

Die akustische Massage der Hörorgane hat eine ähnliche Wirkung. Sie erzielt diese jedoch in einem anderen Frequenzbereich und feiner strukturiert. So fördert auch sie die Durchblutung, wirkt beruhigend auf die Nerven und auf die mentale Verfassung des Patienten. Sie ergänzt die Massagewirkung auf das Innenohr, die durch die Luftdruckmassage erzielt wird, ersetzt diese jedoch nicht.

Durch das Druckgefühl und die akustischen Signale werden zudem die Hörnerven und der akustische Cortex stimuliert, indem die Frequenzbereiche des Tinnitus getroffen werden.

Schließlich fördert das Druckgefühl durch die Luftdruckmassage unter anderem auch die Endorphinausschüttung und somit die mentale Entspannung des Patienten. Sie lenkt diesen auch vom Tinnitusgeräusch ab. Diese Behandlungskomponente ist vor allem bei Stress und bei einer Depressionsneigung des Patienten von besonderer Bedeutung, wie im Rahmen der Erfindung erkannt wurde. Besonders die akustischen Signale wirken hierbei entspannend.

Dabei vereint die erfindungsgemäße Vorrichtung all diese Behandlungskomponenten in einem einzigen Gerät, so dass eine sehr einfache und praktische Anwendung dieser Behandlungsmethode erzielt werden kann. Insbesondere lässt die erfindungsgemäße Vorrichtung eine sehr stressfreie und entspannte Anwendung zu.

Von weiterem Vorteil ist es, dass die erfindungsgemäße Vorrichtung jederzeit mitführbar ist und problemlos durch jeden Anwender bedient werden kann.

Mit der erfindungsgemäßen Vorrichtung wird somit eine ganzheitliche Behandlung von Tinnitus ermöglicht, wodurch sie für praktisch alle Patienten geeignet ist und zuverlässig gute Behandlungserfolge erzielen lässt.

Vorteilhafte Weiterbildungen der erfindungsgemäßen Vorrichtung sind Gegenstand der abhängigen Ansprüche.

So kann die Bedienoberfläche einen Druckregler zum Einstellen der Höhe des Drucks der Druckpulsationen aufweisen. Dann ist es für einen Anwender problemlos möglich, die erfindungsgemäße Vorrichtung gezielt so einzustellen, dass er den besten Behandlungserfolg erreichen kann.

Ferner kann die vom Druckerzeuger erzeugte Druckdifferenz der Druckpulsationen weniger als 0,5 bar betragen. Es hat sich in praktischen Versuchen gezeigt, dass hier noch bessere Ergebnisse erzielbar sind. Dabei kann die Druckdifferenz vorzugsweise auch weniger als 0,2 bar betragen und insbesondere in einem Bereich zwischen 0,05 und 0,1 bar liegen, in dem die besten Behandlungsergebnisse in den Vorversuchen erzielt wurden.

Wenn der Druckerzeuger eine Druckpulsation mit Über- und Unterdruck zum Umgebungsdruck erzeugt, wird eine besonders gute Heilwirkung erreicht. Insbesondere lässt sich dadurch eine besonders gute Massagewirkung und damit Stimulation der Durchblutung etc. erzielen. Auch die Hörnerven und der akustische Cortex lassen sich hierdurch besonders gut stimulieren.

Von weiterem Vorteil ist es, wenn die Bedienoberfläche einen Frequenzregler zum Einstellen der Pulsationsfrequenz der Druckpulsationen aufweist. Dann kann ein Bediener die Pulsationsfrequenz speziell auf den Bereich einstellen, der für ihn individuell den besten Behandlungserfolg erbringt. So ist eine individuelle Anpassung an den jeweiligen Patienten selbständig möglich. In der Praxis wird dabei jedoch jeweils zunächst die geeignete Pulsationsfrequenz des Patienten durch ärztliche Untersuchungen bestimmt und entsprechend voreingestellt. Damit ist eine besonders gezielte und individuelle Behandlung erreichbar.

Wenn die Pulsationsfrequenz weniger als 5 Hz aufweist, lassen sich besonders gute Behandlungsergebnisse erzielen, wie Vorversuche gezeigt haben. Die Pulsationsfrequenz liegt dabei vorzugsweise zwischen 0,25 und 5 Hz, wobei auch hier besonders gute Ergebnisse bei einer Pulsationsfrequenz zwischen 0,5 und 2 Hz erreicht wurden.

Von weiterem Vorteil ist es, wenn die Bedienoberfläche ein Steuerelement zum Einstellen der Lautstärke der Töne und/oder eines bestimmten Tonmusters aus einer Programmmatrix aufweist. Dann ist eine geeignete und individuelle Einstellung der Vorrichtung auf den jeweiligen Patienten möglich, wobei dieser zudem im Zuge der Nutzung noch individuelle Anpassungen vornehmen kann.

Indem die erfindungsgemäße Vorrichtung eine Auswahl an Ohrstöpseln unterschiedlicher Größe und/oder Form aufweist, aus denen der Benutzer den geeigneten Ohrstöpsel wählt, ist diese ein universell vertreibbares Handelsgut. Jeder Nutzer kann dann seine individuell erforderliche Anpassung vornehmen, ohne dass er hierzu zusätzliche Elemente hinzukaufen muss. Dabei ist ein Ohrstöpsel dann besonders geeignet für einen Benutzer, wenn er an die Ohrform bzw. die Gestalt der Ohrmuschel angepasst ist und sich so ein größtmöglicher Tragekomfort bei gleichzeitig optimierter Dichtigkeit ergibt.

Vorteilhaft ist es ferner, wenn die erfindungsgemäße Vorrichtung ein Haltelement aufweist, mittels dem der wenigstens eine Ohrstöpsel im Ohr des Anwenders gehalten wird. Dadurch erhöht sich der Nutzungskomfort weiter. Hierbei ist das Haltelement zudem vorzugsweise auf die Ohr- und/oder Kopfgröße des Anwenders einstellbar. Hierdurch ist nochmals eine Verbesserung des Anwenderkomforts möglich. Zudem wird so eine besonders zuverlässige Funktion der erfindungsgemäßen Vorrichtung erzielt.

Hierbei ist es von Vorteil, wenn das Haltelement ein Kopfhalter ist, welcher den Kopf des Anwenders übergreift; dann ist ein besonders guter Tragekomfort erzielbar. Zudem wird der wenigstens eine Ohrstopsel besonders zuverlässig im Ohr des Anwenders gehalten. Hier kommen vorzugsweise zwei Ohrstöpsel zum Einsatz, so dass beidseitig eine Einwirkung auf das Ohr des Patienten erreicht wird. Damit lassen sich besonders gute Behandlungserfolge erzielen.

Die Erfindung wird nachfolgend in Ausführungsbeispielen anhand der Figuren der Zeichnung näher erläutert. Es zeigt:
- Fig. 1: eine schematische Ansicht der erfindungsgemäßen Vorrichtung;
- Fig. 2: eine Detailansicht im Bereich eines Ohrstöpsels;
- Fig. 3: eine Detailansicht des Anschlussstücks in einem teilweise zusammengebauten Zustand;
- Fig. 4: das Anschlussstück gemäß Fig. 3 im vollständig zusammengebauten Zustand; und
- Fig. 5: das Anschlussstück gemäß Fig. 4 mit aufgesetztem Ohrstöpsel.

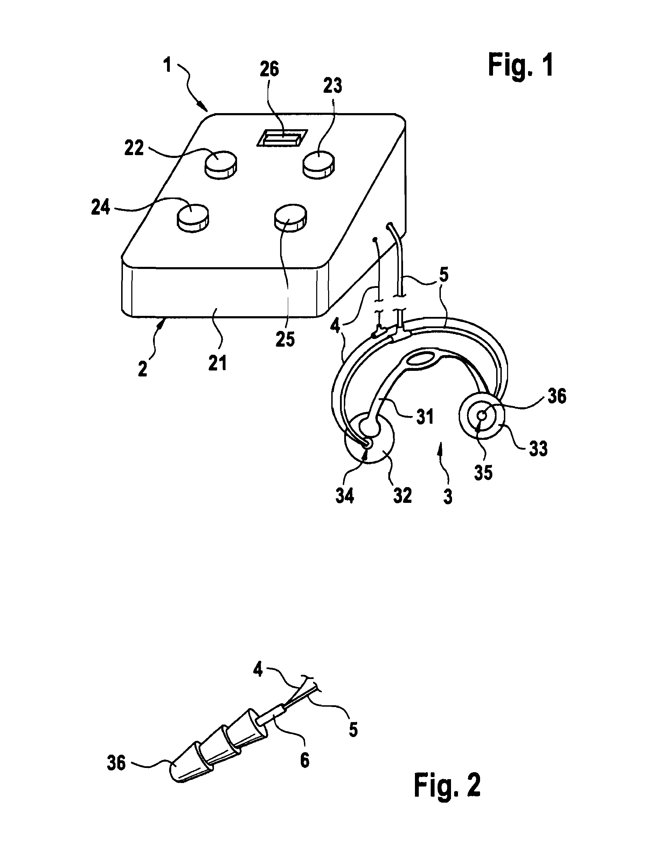

Gemäß der Darstellung in Fig. 1 weist eine Vorrichtung 1 zur Tinnitusbehandlung eine Bedienkonsole 2 sowie einen Kopfhalter 3 auf. Diese sind über ein Lautsprecherkabel 4 und einen Druckschlauch 5 miteinander verbunden.

Die Bedienkonsole 2 weist ein Gehäuse 21 auf, welches hier nur schematisch dargestellt ist. An der Oberseite des Gehäuses 21 befindet sich eine Bedienoberfläche mit mehreren Reglern zur individuellen Einstellung der gewünschten Parameter.

So ist an der Bedienoberfläche ein Druckregler 22 angeordnet, mittels dem die Höhe des Drucks der Druckpulsation einstellbar ist. Im gezeigten Ausführungsbeispiel lässt sich damit eine Druckdifferenz der Druckpulsationen zwischen 0 und 0,5 bar stufenlos einstellen. Dieser Druckregler 22 wirkt dabei mit einem nicht gezeigten Druckerzeuger zusammen, der einen entsprechenden pneumatischen Druck bereitstellt. Der Druckerzeuger ist hier in der Bedienkonsole 2 angeordnet.

An der Bedienoberfläche ist ferner ein Frequenzregler 23 positioniert, mittels dem die Pulsationsfrequenz der Druckpulsationen einstellbar ist. Im gezeigten Ausführungsbeispiel lässt der Frequenzregler 23 eine Pulsationsfrequenz von bis zu 5 Hz zu.

Darüber hinaus ist an der Oberfläche der Bedienkonsole 2 ein Lautstärkeregler 24 angeordnet, mittels dem die Lautstärke der Töne und/oder eines bestimmten Tonmusters aus einer Programmmatrix einstellbar ist.

Hierzu ist ferner ein Programmwahlregler 25 vorgesehen, mittels dem ein Benutzer einen bestimmten Ton oder ein bestimmtes Tonmuster aus einer voreingestellten Programmmatrix frei auswählen kann.

An der Oberfläche der Beginnkonsole 2 ist zudem ein Datenanschluss 26 angeordnet, die hier als USB-Anschluss ausgebildet ist. Hieran kann beispielsweise ein MP3-Player angeschlossen werden, sofern nicht auf voreingestellte Tonmuster der Programmmatrix zugegriffen wird.

Die Bedienkonsole 2 ist dabei so dimensioniert, dass sie problemlos mitgeführt werden kann. Hierzu ist sie mit einem hier nicht dargestellten Clip zur Befestigung des Geräts an einem Gürtel oder dergleichen versehen. Sie ist batteriebetrieben, wobei zusätzlich ein Anschluss für eine direkte Anbindung an ein Stromnetz im normalen Haushaltsbereich gegeben ist. Damit kann die Vorrichtung 1 auch stationär eingesetzt werden bzw. ist ein Wiederaufladen der Batterien bzw. Akkus in der Bedienkonsole 2 möglich.

Aus der Bedienkonsole 2 heraus führen das Lautsprecherkabel 4 und der Druckschlauch 5, welche sich im gezeigten Ausführungsbeispiel jeweils aufteilen und mit den beiden Seiten am Kopfhalter 3 verbunden sind. Dieser weist einen Haltebügel 31 sowie Ohrmuscheln 32 und 33 auf. Der Kopfhalter 3 ist insofern zunächst ähnlich einem herkömmlichen Kopfhalter für Musikanlagen oder dgl. ausgebildet. Allerdings sind die Ohrmuscheln 32 und 33 so ausgestaltet, dass sie spezielle Stopselaufnahmen 34 und 35 enthalten. In diese können Ohrstöpsel 36 eingefügt werden, von denen in Fig. 1 nur einer gezeigt ist. Diese Ohrstöpsel 36 sind dabei in den Ohrmuscheln 32 und 33 arretiert, so dass sie in geeigneter Weise im äußeren Gehörgang des Ohres zu liegen kommen.

Aus Fig. 2 ist ersichtlich, dass ein solcher Ohrstöpsel 36 über ein Anschlussstück 6 mit dem Druckschlauch 5 sowie dem Lautsprecherkabel 4 verbunden ist. Der Ohrstöpsel 36 ist dabei so ausgebildet, dass der durch den Druckschlauch 5 zugeführte Luftdruck durch diesen hindurch in den äußeren Gehörgang hinein wirken kann. Dabei ist der Druckschlauch 5 so ausgestaltet, dass er flexibel ist und sich bei dem angewandten Druck im Wesentlichen nicht dehnt.

Zur Anwendung der Vorrichtung 1 zur Tinnitusbehandlung wählt ein Benutzer für ihn geeignete Ohrstöpsel 36 aus, befestigt diese an den Stöpselaufnahmen 34 und 35 und verbindet diese mit dem Anschlussstück 6 mit dem Lautsprecherkabel 4 und dem Druckschlauch 5. Dann setzt er den Kopfhalter 3 auf. Anschließend schaltet er die Vorrichtung 1 mittels einem nicht dargestellten Schalter ein und wählt das gewünschte Programm aus der Programmmatrix mittels dem Programmwahlregler 25. Mit Hilfe des Druckreglers 22, des Frequenzreglers 23 und des Lautstärkereglers 24 nimmt er dann individuelle Anpassungen dieser Parameter vor, um so den Behandlungserfolg zu optimieren.

Damit erhält ein Benutzer eine Luftdruckmassage sowie auch eine akustische Massage der Hörorgane. Ferner werden die Hörnerven und der akustische Cortex stimuliert und es stellt sich eine mentale Entspannung ein.

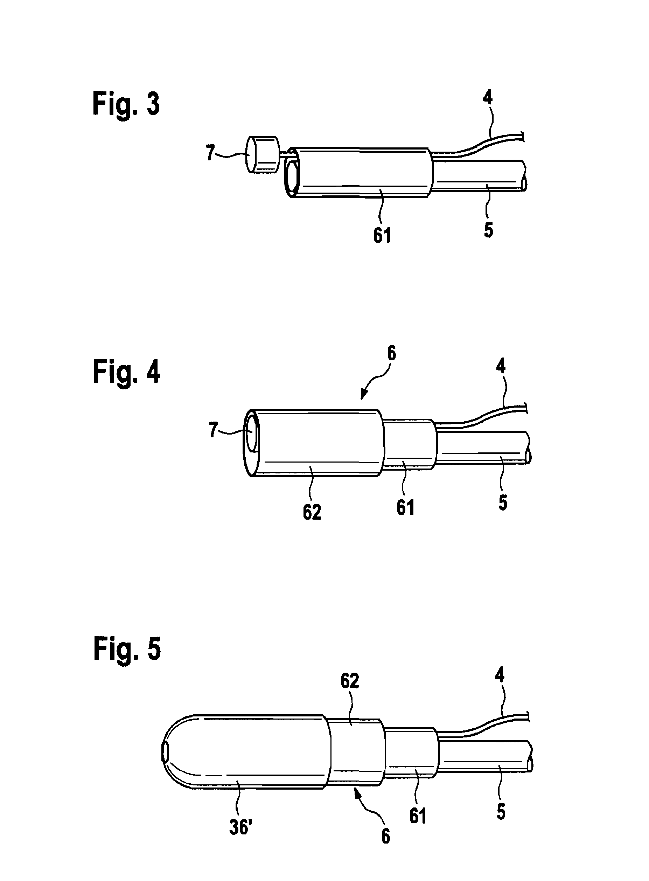

In den Fig. 3 bis 5 ist der Bereich des Anschlussstücks 6 in näherem Detail gezeigt. Fig. 3 zeigt hierbei einen teilweise zusammengesetzten Zustand des Anschlussstücks 6. Zu erkennen ist hier, dass das Lautsprecherkabel 4 in einen Lautsprecher 7 mündet. Dieser weist eine besonders geringe Größe auf, damit er für den vorgesehenen Einsatzfall geeignet ist. Der Druckschlauch 5 mündet hier mit offenem Ende neben dem Lautsprecherkabel 4 im Bereich des Lautsprechers 7. Das Lautsprecherkabel 4 und der Druckschlauch 5 sind dabei durch einen Schrumpfschlauch 61 miteinander verbunden.

In der Darstellung gemäß Fig. 4 ist über den Schrumpfschlauch 61 und den Lautsprecher 7 zudem noch ein Stülpschlauch 62 aufgesetzt, so dass das Anschlussstück 6 fertig ausgebildet ist. Sowohl der Lautsprecher 7 als auch das offene Ende des Druckschlauchs 5 liegen somit dann am freien Ende des Anschlussstücks 6 in geeigneter Weise vor. Hierauf ist schließlich gemäß der Darstellung in Fig. 5 ein medizinischer Ohrstöpsel 36' aufgesetzt, welcher anders als der in Fig. 2 gezeigte Ohrstöpsel 36 ausgebildet ist. Diese Anordnung kann nun so in eine der Stöpselaufnahmen 34 bzw. 35 am Kopfhalter 3 eingesetzt werden.

Die erfindungsgemäße Vorrichtung 1 zur Tinnitusbehandlung lässt neben der erläuterten Ausführungsform weitere Gestaltungsansätze zu.

So ist es in einer vereinfachten Ausführungsform auch möglich, auf den Druckregler 22 zu verzichten. Die entsprechende Vorrichtung arbeitet dann immer mit dem werkseitig eingestellten Druck der Druckpulsation.

Ferner kann es in besonderen Anwendungsfällen auch sachgerecht sein, die Druckdifferenz der Druckpulsation mit einem höheren Wert als 0,5 bar vorzusehen. Hier sind die individuellen Bedürfnisse der zu behandelnden Person zu berücksichtigen. Grundsätzlich kann es in besonderen Anwendungsfällen auch sachgerecht sein, eine Druckdifferenz von weniger als 0,05 bar einzusetzen.

In einer bevorzugten Ausführungsform der Erfindung stellt der Druckerzeuger eine Druckpulsation mit Über- und Unterdruck zum Umgebungsdruck bereit. Dies ist jedoch nicht erforderlich. Die Vorrichtung 1 kann auch ausschließlich im Unter- oder im Überdruckbereich zum Umgebungsdruck betrieben werden. Dann entsteht die Druckpulsation durch Druckaufbau und Druckablass durch den Druckerzeuger.

In einer weiteren vereinfachten Ausgestaltungsweise kann die Vorrichtung 1 auch ohne den Frequenzregler 23 eingesetzt werden. Dann kommt eine werkseitig vorab eingestellte Pulsationsfrequenz zur Anwendung.

Auch im Hinblick auf die Pulsationsfrequenz ist es im Lichte der sehr unterschiedlichen von einem Tinnitus betroffenen Menschen möglich, hier einen Wert von mehr als 5 Hz zur Anwendung zu bringen, falls dies im Einzelfall sachgerecht sein sollte.

Zudem ist es auch möglich, auf den Lautstärkeregler 24 im Rahmen einer vereinfachten Ausgestaltungsweise der Vorrichtung 1 zu verzichten. Dann kommt eine werkseitig vorab eingestellte Lautstärke zum Tragen.

Auch der Programmwahlregler 25 ist bei einer vereinfachten Variante der Vorrichtung verzichtbar. Dann kommt ein werkseitig voreingestellter Ton bzw. ein bestimmtes voreingestelltes Tonmuster zur Anwendung.

Wie aus diesen Erläuterungen ersichtlich ist, können einzelne oder mehrere oder auch alle Regler 22 bis 25 an der Bedienkonsole 2 entfallen. In der Ausführungsform gemäß Fig. 1 werden jedoch sämtliche Regler 22 bis 25 eingesetzt.

Ferner ist es auch möglich, ein Tonmuster von den unterschiedlichsten externen Quellen einzusetzen. Dies ist nicht auf den oben angesprochenen MP3-Player beschränkt, sondern kann auch andere Datenquellen wie USB-Sticks, Internetverbindungen oder dgl. nutzen, wobei zudem auch der Datenanschluss 26 für andere Datenanschlusssysteme als USB ausgerüstet sein kann wie z.B. SD etc.. Alternativ ist es allerdings auch möglich, auf den Datenanschluss 26 zu verzichten.

In einer alternativen Ausgestaltungsweise ist es ferner auch möglich, statt einer einzelnen Bedienkonsole 2 zwei oder mehrere separate Elemente einzusetzen. So kann der Druckerzeuger mit seinen Steuerungselementen eine separate Einheit bilden, wie auch die Tonquelle mit seinen zugeordneten Steuerungselementen. Wesentlich ist hierbei nur, dass im Ohrbereich des Anwenders sowohl der Druck als auch die Tonanwendung zugleich bereit stehen und zur Wirkung kommen können.

In einer bevorzugten Ausführungsform weist die Vorrichtung 1 eine Auswahl an Ohrstöpseln in unterschiedlicher Größe und/oder Form auf, woraus der Benutzer den geeigneten Ohrstöpsel wählen kann. Dies ist jedoch nicht zwingend erforderlich. Hier kann auch ein Standard-Ohrstöpsel zum Einsatz kommen.

Dabei ist es in einer vereinfachten Ausführungsform der Vorrichtung 1 nicht erforderlich, dass ein Kopfhalter 3 vorgesehen ist. Außerdem kann ein einzelner Ohrstöpsel 36 hinreichend sein, wobei dies insbesondere dann der Fall ist, wenn der Tinnitus beim Anwender nur einseitig auftritt. Dann kann dieser einzelne Ohrstöpsel 36 ohne Verwendung einer Ohrmuschel 32 bzw. 33 oder eines Haltebügels 31 direkt in das Ohr eingefügt werden.

Andererseits kann die Ausgestaltung bei Anwendung des Haltebügels 31 mit Ohrmuscheln 32 und 33 so getroffen sein, dass diese Elemente individuell auf die Kopfform anpassbar sind, wie dies an sich bereits aus dem Bereich von Musikanlagen bekannt ist. Ferner können auch Haltebügel in unterschiedlichen Größen angeboten werden.

Zudem ist es bei einer einseitig gewünschten Anwendung der Vorrichtung 1 auch möglich, den Haltebügel 31 nur einseitig mit einem Ohrstöpsel 36 zu bestücken, so dass die Anwendung dann nur gezielt an dem einen betroffenen Ohr erfolgt. Andererseits ist es hier auch möglich, die beiden Ohren des Benutzers nicht gleichzeitig, sondern abwechselnd zu behandeln, was für bestimmte Formen von Tinnitus vorteilhaft ist. Letztendlich ist hier somit das spezielle Krankheitsbild des Anwenders maßgeblich für die jeweilige Einsatzart der Vorrichtung 1.

Zur Unterstützung der Behandlung ist es ferner auch möglich, den Einsatz der Vorrichtung 1 mit der Einnahme oder Infusion von medizinischen Substanzen oder mit stressmindernden Maßnahmen zu kombinieren.

## Patentansprüche

1. Vorrichtung (1) zur Tinnitusbehandlung, mit einer Bedienkonsole (2) mit einer Bedienoberfläche und mit wenigstens einem mit der Bedienkonsole (2) verbundenen Ohrstöpsel (36; 36') zur Einbringung eines Tonmusters aus Tönen mit Tonhöhen in einer Frequenz von mehr als 30 Hz in den äußeren Gehörgang eines Ohres,
**dadurch gekennzeichnet,**
**dass** der Ohrstöpsel (36; 36') über einen Druckschlauch (5) mit einem Druckerzeuger verbunden ist, der Druckpulsationen mit einer Pulsationsfrequenz von weniger als 10 Hz und einer Druckdifferenz von weniger als 1 bar erzeugt und über den Druckschlauch (5) in den äußeren Gehörgang des Ohres zuführt.

2. Vorrichtung nach Anspruch 1, **dadurch gekennzeichnet, dass** die Bedienoberfläche einen Druckregler (22) zum Einstellen der Höhe des Drucks der Druckpulsationen aufweist.

3. Vorrichtung nach Anspruch 1 oder 2, **dadurch gekennzeichnet, dass** die vom Druckerzeuger erzeugte Druckdifferenz der Druckpulsationen weniger als 0,5 bar, vorzugsweise weniger als 0,2 bar, und insbesondere zwischen 0,05 und 0,1 bar, beträgt.

4. Vorrichtung nach einem der Ansprüche 1 bis 3, **dadurch gekennzeichnet, dass** der Druckerzeuger eine Druckpulsation mit Über- und Unterdruck zum Umgebungsdruck erzeugt.

5. Vorrichtung nach einem der Ansprüche 1 bis 4, **dadurch gekennzeichnet, dass** die Bedienoberfläche einen Frequenzregler (23) zum Einstellen der Pulsationsfrequenz der Druckpulsationen aufweist.

6. Vorrichtung nach einem der Ansprüche 1 bis 5, **dadurch gekennzeichnet, dass** die Pulsationsfrequenz weniger als 5 Hz, vorzugsweise zwischen 0,25 und 5 Hz, und insbesondere zwischen 0,5 und 2 Hz aufweist.

7. Vorrichtung nach einem der Ansprüche 1 bis 6, **dadurch gekennzeichnet, dass** die Bedienoberfläche ein Steuerelement (24, 25) zum Einstellen der Lautstärke der Töne und/oder eines bestimmten Tonmusters aus einer Programmmatrix aufweist.

8. Vorrichtung nach einem der Ansprüche 1 bis 7, **dadurch gekennzeichnet, dass** sie eine Auswahl von Ohrstöpseln (36; 36') unterschiedlicher Größe und/oder Form aufweist, woraus der Benutzer den geeigneten Ohrstöpsel (36; 36') wählt.

9. Vorrichtung nach einem der Ansprüche 1 bis 8, **dadurch gekennzeichnet, dass** sie ferner ein Halteelement aufweist, mittels dem der wenigstens eine Ohrstöpsel (36; 36') im Ohr des Anwenders gehalten ist, wobei das Halteelement vorzugsweise auf die Ohr- und/oder Kopfgröße des Anwenders einstellbar ist.

10. Vorrichtung nach Anspruch 9, **dadurch gekennzeichnet, dass** das Halteelement ein Kopfhalter (3) ist, welcher einen Kopf des Anwenders übergreift, wobei vorzugsweise zwei Ohrstöpsel (36; 36') zum Einsatz kommen.
